# EUROPEAN PATENT SPECIFICATION

(11) **EP 2 814 549 B1**
(45) Date of publication and mention of the grant of the patent: **21.12.2016**
(21) Application number: 13707043.9
(22) Date of filing: 16.01.2013
(51) Int. Cl.: A61M 5/46

(54) **DEVICE FOR THE CONTROL OF THE PENETRATION DEPTH OF A NEEDLE FOR INJECTIONS**
VORRICHTUNG ZUR STEUERUNG EINER NADELEINSTICHTIEFE FÜR INJEKTIONEN
DISPOSITIF DE COMMANDE DE LA PROFONDEUR DE PÉNÉTRATION D'UNE AIGUILLE D'INJECTION

(30) Priority: 16.02.2012 IT MO20120040
(43) Date of publication of application: 24.12.2014
(73) Proprietor: Lameplast S.p.A., 41016 Rovereto sul Secchia - Novi Di Modena (MO) (IT)
(72) Inventor: FONTANA, Antonio, I-41012 Carpi (MO) (IT)
(74) Representative: Brunacci, Marco
(86) International application number: PCT/IB2013/050390
(87) International publication number: WO 2013/121307

(56) References cited:
- EP-A1- 1 045 710
- WO-A1-02/04053
- US-A1- 2006 229 562

## Description

### Technical Field

The present invention relates to a device for the control of the penetration depth of a needle for injections.

### Background Art

As is known, on various occasions, the need arises to inject fluid substances inside the human body by means of injection devices such as syringes or the like.

Depending on the type of fluid to be injected and on the treatment to be applied, the injections can involve the surface layer of the skin only and/or the layers underneath.

In particular, for the surface, so-called "intraepidermal" injections, the syringe needle must penetrate the skin to a very limited depth.

Nevertheless, the syringes of traditional type are not particularly efficient in this respect inasmuch as reaching or not the correct depth of needle penetration depends on the manual skill of the person performing the injection.

To this must be added that an incorrect needle penetration depth can cause, on the one hand, unpleasant feelings of pain by the patient and, on the other hand, an incomplete absorption of the injected substance.

It is in fact underlined that surface injections are easily subject to the leaking out of the injected substance as soon as the injection needle is removed.

Furthermore, it must be observed how treatments based on intraepidermal injections such as, in particular, those of dermatological type, require a high number of intraepidermal injections, with the recurrence each time of the aforementioned problems.

To overcome the aforementioned drawbacks, at least in part, devices are known like those shown in the patent document EP 1 045 710, wherein an element is devised meant to come into contact with the skin that can be assembled around the injection needle.

The contact element has a hollow overturned-cone portion which terminates with a circular edge.

During the injection, the circular edge is the first to come into contact with the patient's skin, producing its deformation, which allows the needle to penetrate inside to a predetermined extent.

Once the injection has been completed, the moving away of the syringe permits the patient's skin to return elastically to its original shape without the injected fluid coming out and thus favouring absorption.

This device of known type is also not without its drawbacks however.

In this respect, it must be emphasized that the human skin, and above all its outermost layer, is rather discontinuous and uneven in terms of elasticity, resistance to needle penetration and resistance to deformation, and consequently behaves in different ways according to the point of the body in which the injection is made and according to the type of skin, which changes considerably from one patient to another.

In the light of such considerations, it appears evident that the device according to the patent EP 1 045 710 does not always allow the needle to reach the pre-established depth due to the different deformation of the human skin.

On the other hand, the possibility of having a plurality of devices according to the patent EP 1 045 710, distinguished by a different needle penetration depth, would not be very practical inasmuch as it would require the use of an excessive number of needles.

In this respect, it must be underlined that traditional needles for intraepidermal injections which do not make use of the device shown in patent EP 1 045 710 are usable several times on the same patient and, usually, no more than one per session is used.

It therefore follows that using a plurality of devices according to the patent EP 1 045 710 in just one therapy session would represent a considerable waste and a far-from-negligible cost.

The device shown in EP 1 045 710, furthermore, is also susceptible to upgrading as regards the possibility of reducing the pain caused by the penetration of the needle.

To all this must be added that, inconveniently, the assembly of the contact element and of the injection needle must necessarily occur during the production and packaging phases of the products which must then be sanitized and sterilized before being placed on the market.

In this respect, it is underlined that the injection needles used for the manufacture of these devices are needles already present on the market which, therefore, had already been sanitized and sterilized in a previous stage of manufacture before being assembled with the contact element and which undergo further sanitizing after assembly.

All this represents a considerable complication of the production process and a considerable increase in manufacturing costs which, inevitably, affect the retail price, with the risk of making the product less appealing for consumers.

A device according to the preamble of claim 1 is disclosed in the patent document WO 02/04053.

### Description of the Invention

The main aim of the present invention is to provide a device for the control of the penetration depth of a needle for injections which permits making injections in a practical, easy and functional way in any point of the human body and whatever the type of skin of the patient.

A further object of the present invention is to permit making several injections on the same patient without having to change needle.

Not the last object of the present invention is to considerably simplify the process of manufacture and market distribution, thus making it possible to cut costs and the retail price to consumers.

Another object of the present invention is to provide a device for the control of the penetration depth of a needle for injections which allows to overcome the mentioned drawbacks of the state of the art in the ambit of a simple, rational, easy and effective to use as well as low cost solution.

The above objects are achieved by the present device for the control of the penetration depth of a needle for injections having the features of claim 1.

### Brief Description of the Drawings

Other characteristics and advantages of the present invention will become more evident from the description of a preferred, but not sole, embodiment of a device for the control of the penetration depth of a needle for injections, illustrated purely as an example but not limited to the annexed drawings in which:
Figure 1 is an exploded view of an injection needle, a syringe and of the device according to the invention to be used together with the injection needle and the syringe;

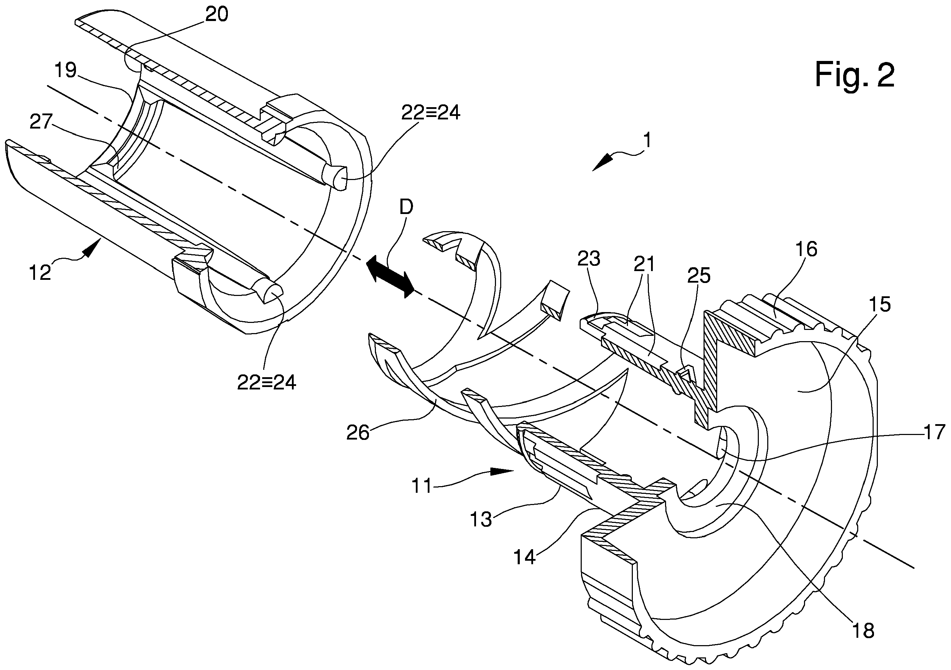

Figure 2 is an exploded, broken view on enlarged scale of the device according to the invention;
Figures from 3 to 5 show, in a series of section views, the operation of the device according to the invention.

### Embodiments of the Invention

With particular reference to such figures, globally indicated by 1 is a device for the control of the penetration depth of a needle for injections.

The device 1 is intended to be used together with a dispensing device 2 for dispensing a substance F, such as a syringe or the like, and with an injection needle 3 for injecting the substance F in the skin P of a user, e.g., a patient undergoing an intraepidermal injection treatment.

The syringe 2 and the injection needle 3 are of the known type.

The syringe 2, in particular, comprises a syringe body 4 for containing the substance F, wherein is inserted a thrust piston 5 in a sliding and sealed way.

One extremity of the syringe body 4 terminates in a neck 6 for attaching the injection needle 3.

For this purpose, the injection needle 3 comprises a tubular element 7 having an open extremity which can be interlocked over the neck 6 of the syringe 2.

The outer edge of the open extremity has a pad 8.

In correspondence to the opposite extremity of the tubular element 7 is engaged a point 9 for penetration into the skin P of the patient.

The injection needle 3 is intended to be coupled with a cap 10 which can be fitted on the tubular element 7 to cover the point 9.

The substance F to be injected with the syringe 2 is in fluid product state; in this respect, it is pointed out that within the scope of this treatise, by the term fluid product is not only meant liquid products but also viscous products, e.g. in the state of paste or gel.

The device 1 comprises at least a supporting element 11, which is associable with at least one between the syringe 2 and the injection needle 3.

This means that the supporting element 11 can be:
- fitted on the syringe 2, e.g., on the neck 6 or on the syringe body 4, without coming into direct contact with the injection needle 3;
- fitted on the injection needle 3, e.g., on its outer surface, without coming into direct contact with the syringe 2;
- placed between the syringe 2 and the injection needle 3, i.e., it can be fitted on the syringe 2 and in turn support the injection needle 3.

In the embodiment shown in the illustrations, the supporting element 11 is intended to be fitted on the injection needle 3, which in turn is fitted over the neck 6 of the syringe 2, as is better described below.

To the supporting element is associable at least one contact element 12, which can translate with respect to the supporting element 11 along a sliding direction D substantially parallel to the injection needle 3 and suitable for coming into contact with the skin P of the user.

Advantageously, the supporting element 11 is defined by a first round-section tubular body, while the contact element 12 is defined by a second round-section tubular body.

The tubular bodies 11, 12 are fitted coaxially one inside the other, are mobile in a telescopic way and at least partially surround the injection needle 3.

More in detail, the first tubular body 11 has a first portion 13 fitted in the second tubular body 12 and a second portion 14 exiting from the second tubular body 12.

In correspondence to the second portion 14, the first tubular body 11 comprises a widened manoeuvre portion 15.

The widened manoeuvre portion 15 consists, for example, of a disc with diameter considerably bigger than the first tubular body 11 and having a grooved edge 16 for easier grip by the operator in charge of operating the syringe 2.

The supporting element 11 comprises interlocking means 17 of the injection needle 3.

The interlocking means 17 comprise one or more tooth elements which are obtained on the inner surface of the first tubular body 11 and are interlockable on a corresponding locator obtained on the injection needle 3.

Advantageously, the locator of the injection needle 3 in which the tooth elements 17 are interlockable consists in the pad 8.

In interlock position, the pad 8 remains blocked between the tooth elements 17 and an annular bottom 18 extending inside the first tubular body 11.

The annular bottom 18 is open to allow the transit of the neck 6 of the syringe 2 and its interlock coupling with the inner surface of the injection needle 3.

Both the extremities of the second tubular body 12 are open.

More in detail, one open extremity is turned towards the supporting element 11 and is crossed by the first tubular body 11.

The other open extremity, instead, is intended to be rested on the skin P of the patient and to circumscribe the area of the derma in correspondence to which the injection is made.

The edge 19, 20 of this latter open extremity is defined by a series of rounded sections 19 alternated with a series of sharp-edge sections 20.

The sharp-edge sections 20 produce a sort of "pinch effect" on the skin P of the patient which relieves the pain produced by the jab of the injection needle 3.

The diameter of the first tubular body 11 and of the second tubular body 12 is sized so as to allow the insertion of the injection needle 3 and of the cap 10 from the opposite side with respect to the annular bottom 18.

Usefully, the device 1 comprises adjustment means 21, 22 suitable for making it possible to set the travel stroke of the contact element 12 on the supporting element 11.

In this respect, it is underlined that the travel stroke of the contact element 12 is defined between a first end-of-stroke position, corresponding to the maximum elongation of the contact element 12 on the supporting element 11, and a second end-of-stroke position, corresponding to the minimum elongation of the contact element 12 on the supporting element 11.

The adjustment means 21, 22 are suitable for modifying the second end-of stroke position, while the first end-of-stroke position remains substantially unchanged in all work configurations.

In this respect, it is pointed out that the supporting element 11 comprises at least a first locator tooth 23 and the contact element 12 comprises at least a second locator tooth 24.

The locator teeth 23, 24 are suitable for coming into contact in the first end-of-stroke position and preventing the complete removal of the contact element 12 from the supporting element 11.

The first locator tooth 23 has a substantially annular shape and is obtained on the outer surface of the first portion 13, in correspondence to the extremity of the first tubular body 11 turned towards the contact element 12.

On the contact element 12, instead, a plurality of second locator teeth 24 are defined, which are obtained on the inner surface of the second tubular body 12, in correspondence to the extremity turned towards the supporting element 11. The adjustment means 21, 22 comprise:
- at least a group of guiding grooves 21, which are obtained on one between the supporting element 11 and the contact element 12 and extend substantially parallel to the sliding direction D; and
- at least a guiding pin 22, which is associated with the other between the supporting element 11 and the contact element 12 and can be alternatively fitted in a sliding way in one of the guiding grooves 21.

The guiding grooves 21 have different lengths to define different travel strokes. More in detail, the guiding grooves 21 are obtained on the outer surface of the first portion 13 of the first tubular body 11, while the guiding pin 22 is associated with the inner surface of the second tubular body 12.

Alternative embodiments cannot however be ruled out wherein the guiding grooves 21 are obtained on the inner surface of the second tubular body 12 while the guiding pin 22 is associated with the outer surface of the first tubular body 11.

The guiding grooves 21 are substantially arranged one alongside the other and the tubular bodies 11, 12, by virtue of their round section, are rotatable around their axes to force the passage of the guiding pin 22 from one guiding groove 21 to the other.

In other words, the travel stroke of the contact element 12 on the supporting element 11 is defined by the length of the guiding groove 21 in which the guiding pin 22 is inserted; by reciprocally rotating the tubular bodies 11, 12 around their axes, the guiding pin 22 is forced to exit from a guiding groove 21 and engage an adjacent one.

Advantageously, the adjustment means 21, 22 comprise a plurality of groups of guiding grooves 21 and a corresponding plurality of guiding pins 22, one for each group of guiding grooves 21.

Each guiding groove 21 has a length that is different from the length of the grooves of the same group but is the same as the length of a guiding groove 21 of each of the other groups.

The guiding pins 22 are distributed around the second tubular body 12 with a constant pitch; in the same way, the groups of guiding grooves 21 are distributed around the first tubular body 11 with a constant pitch.

In the embodiment shown in the illustrations, for example, there are four guiding pins out of phase by 90° the one from the other.

In the same way, there are four groups of guiding grooves 21 out of phase by 90° the one from the other; this means that the guiding grooves 21 of the same length are out of phase by 90° the one from the other.

Each group of guiding grooves 21 is composed of four guiding grooves 21, for a total of sixteen guiding grooves 21 distributed around the first tubular body 11.

The four guiding grooves 21 of each group have a length substantially equal to 1 mm, 2 mm, 3 mm and 4 mm respectively, and are distinguished by a corresponding number 25, shown or shaped on the outer surface of the first tubular body 11.

The numbers 25 allow the operator in charge of injection to identify in a practical and easy way the work configuration being operated in.

The particular solution of providing four guiding pins 22 and four groups of guiding grooves 21 distributed around the tubular bodies 11, 12 permits stabilizing the reciprocal sliding movement of the supporting element 11 and of the contact element 12.

Alternative embodiments cannot however be ruled out wherein the guiding pins 22 and, correspondingly, the groups of guiding grooves 21, are of different number, e.g., three pins out of phase by 120°, or two pins out of phase by 180°, or also just one guiding pin 22.

At the same time, it is underlined that the particular solution of providing four guiding grooves 21 for each group permits providing the device 1 with four work configurations distinguished by four different travel strokes among which to choose the one most suitable according to use.

In this case as well however, alternative embodiments cannot be ruled out wherein each group is equipped with a different number of guiding grooves 21 and with corresponding work configurations.

It must be noted, furthermore, that in the embodiment shown in the illustrations, the guiding pins 22 and the second locator teeth 24 coincide with one another.

In other words, the guiding pins 22 protrude on the inner surface of the second tubular body 12, engaging the guiding grooves 21 and, in the first end-of-stroke position, stop up against the first locator tooth 23 performing the aforementioned functions of the second locator teeth 24.

Different embodiments are however possible wherein the guiding pins 22 and the second locator teeth 24 are distinct the one from the other.

Finally, the adjustment means 21, 22 comprise return flexible means 26 suitable for pushing the contact element 12 to return to the first end-of-stroke position. In the embodiment shown in the illustrations, the return flexible means 26 comprise a section of the first tubular body 11 which has a helical-spring shape and an extremity stopped up against a protrusion 27 of the second tubular body 12.

Alternative embodiments cannot however be ruled out wherein, on the contrary, the return flexible means 26 are defined by a section of the second tubular body 12 which has a helical-spring shape and an extremity stopped up against a protrusion of the first tubular body 11.

The operation of the device 1 is the following.

In the production phase, the supporting element 11 and the contact element 12 are made separately, e.g., by means of plastic injection moulding.

Subsequently, they are sanitized, assembled by inserting the first tubular body 11 inside the second tubular body 12, and packed in sterile conditions.

The device 1 is distributed on the market separately from the syringe 2 and from the injection needle 3, which are sanitized and packed separately.

At the time of use, the operator charged with making the injection fits the injection needle 3 inside the device 1.

For this purpose, it must be underlined that the cap 10 covers the entire injection needle 3 except for the pad 8 and can be used to insert the injection needle 3 inside the tubular bodies 11, 12 in conditions of utmost safety and hygiene.

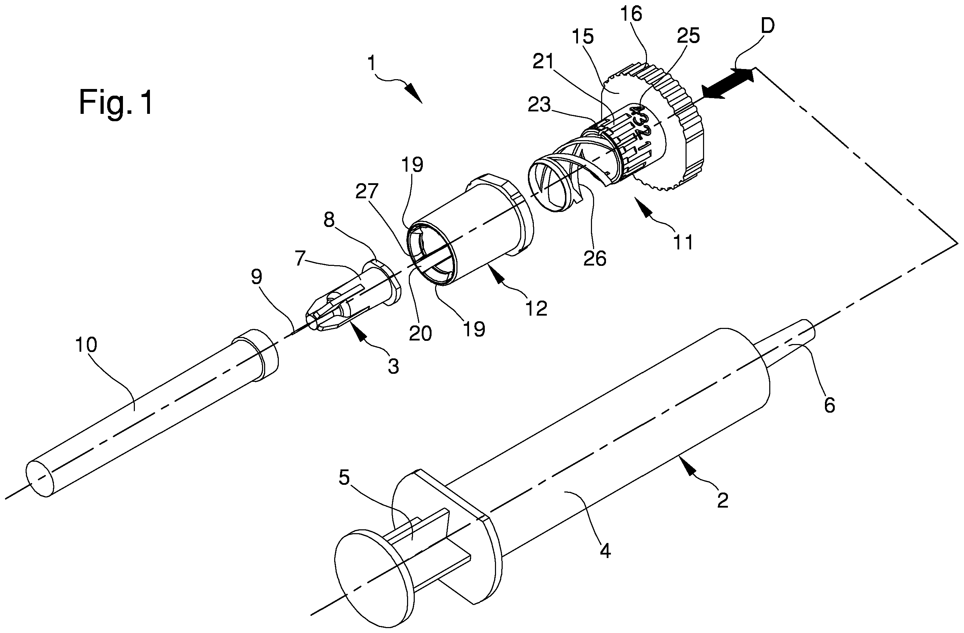

The operator can in fact handle the injection needle 3 without touching it directly but simply gripping the cap 10 and introducing it inside the tubular bodies 11, 12 until the pad 8 is blocked in the tooth elements 17, as can be seen in the figure 1.

At this point, the cap 10 can be removed from the injection needle 3, which remains fitted on the device 1.

The injection needle 3 and the device 1 are then fitted on the syringe 2 blocking the inner surface of the tubular element 7 on the neck 6.

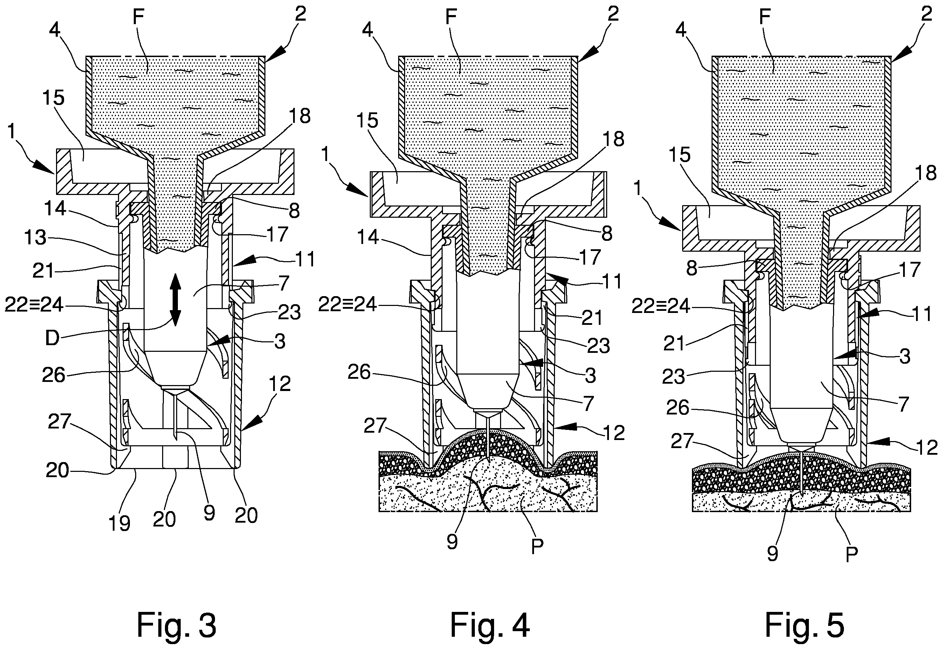

In this configuration, the device 1 has the tubular bodies 11, 12 arranged around the injection needle 3, with the return flexible means 26 which push the contact element 12 in the first end-of-stroke position and the guiding pins 22 which are stopped up against the first locator tooth 23 (figure 3).

At this point, by making the tubular bodies 11, 12 reciprocally rotate around their axes, the guiding pins 22 are forced to pass from one guiding groove 21 to another, modifying the travel stroke of the device 1.

For this purpose, the operator can firmly grip the supporting element 11 in correspondence to the widened manoeuvre portion 15 and the contact element 12 in correspondence to the outer surface of the second tubular body 12.

The choice of the work configuration of the device 1 is made according to the injection depth to be reached in accordance with the elasticity and the resistance of the skin P, which varies according to the point of the body where the injection is made and to the type of skin of the patient.

During the injection, in fact, the edge 19, 20 of the second tubular body 12 comes into contact with the skin P of the patient and causes its deformation which permits the injection needle 3 to penetrate inside to a predetermined extent.

By way of example only, the figures 4 and 5 show the device 1 in the second end-of-stroke position corresponding to two different travel strokes wherein, by virtue of a different behaviour of the skin P of the patient, the injection needle 3 substantially reaches the same penetration depth.

In figure 4, in fact, the skin P of the patient is very soft and deforms in a heavily accented way; to reach the preset depth, a minimum travel stroke is enough (notice the short length of the guiding grooves 21 in which the guiding pins 22 are inserted).

In figure 5, instead, the skin P of the patient is very rigid and not very deformable; in this case, to reach the same penetration depth, a greater travel stroke is needed (the length of the guiding grooves 21 is greater).

In a completely similar way, the device 1 can also allow making injections at different depths, always taking into account the deformability and the behaviour of the skin P.

It has in fact been ascertained how the described invention achieves the proposed objects.

In particular, it is underlined that with the present device, the penetration depth of the needle no longer depends on the skill of the operator, but is set before making the injection by rotating the supporting element and the contact element with respect to one another.

Thanks to the present device, furthermore, the same injection needle can be reused several times on the same patient, selecting a different travel stroke each time.

It is further underlined that the device according to the present invention can be manufactured in a practical and easy way.

The device is in fact placed on the market separately from the injection needle and from the syringe, assigning the task of assembling them to the end operator.

The device is furthermore made and sized so as to be compatible with the traditional injection needles on the market, favouring its use among operators in the sector.

## Claims

1. Device (1) for the control of the penetration depth of a needle for injections, comprising:
- at least a supporting element (11), which is associable with at least one between a dispensing device (2) for dispensing a substance (F) and an injection needle (3) for the injection of the substance (F) in the skin (P) of a user;
- at least a contact element (12), which is associable with said supporting element (11) in a translatable way along a sliding direction (D) substantially parallel to said injection needle (3) and suitable for coming into contact with the skin (P) of the user ; and
- adjustment means (21, 22) for adjusting the travel stroke of said contact element (12) on said supporting element (11);
**characterized by** the fact that said supporting element (11) comprises interlocking means (17) of said injection needle (3) which comprise at least a tooth element which can be interlocked on a corresponding locator obtained on said injection needle (3), said supporting element (11) is intended to be fitted on said injection needle (3), which in turn is fitted over said dispensing device (2).

2. Device (1) according to claim 1, **characterized by** the fact that said travel stroke is defined between a first end-of-stroke position, corresponding to the maximum elongation of said contact element (12) on said supporting element (11), and a second end-of-stroke position, corresponding to the minimum elongation of said contact element (12) on said supporting element (11), said adjustment means (21, 22) being suitable for modifying said second end-of-stroke position.

3. Device (1) according to claim 2, **characterized by** the fact that said adjustment means (21, 22) comprise return flexible means (26) suitable for pushing said contact element (12) to return into said first end-of-stroke position.

4. Device (1) according to claim 2 or 3, **characterized by** the fact that said supporting element (11) comprises at least a first locator tooth (23) and said contact element (12) comprises at least a second locator tooth (24), said locator teeth (23, 24) being suitable for coming into contact in said first end-of-stroke position and preventing the complete removal of said contact element (12) from said supporting element (11).

5. Device (1) according to one or more of the preceding claims, **characterized by** the fact that said supporting element (11) comprises a first tubular body and said contact element (12) comprises a second tubular body, wherein said tubular bodies (11, 12) are fitted telescopically one inside the other and at least partially surround said injection needle (3).

6. Device (1) according to claim 5, **characterized by** the fact that said first tubular body (11) comprises a first portion (13) fitted in said second tubular body (12) and a second portion (14) exiting from said second tubular body (12).

7. Device (1) according to claim 6, **characterized by** the fact that said first tubular body (11) comprises at least a widened manoeuvre portion (15) associated with said second portion (14).

8. Device (1) according to one or more of the preceding claims, **characterized by** the fact that said adjustment means (21, 22) comprise:
- at least a group of guiding grooves (21), which are obtained on one between said supporting element (11) and said contact element (12) and extend substantially parallel to said sliding direction (D); and
- at least a guiding pin (22), which is associated with the other between said supporting element (11) and said contact element (12) and can be alternatively fitted in a sliding way into one of said guiding grooves (21);
said guiding grooves (21) having different lengths to define different travel strokes.

9. Device (1) according to claim 8, **characterized by** the fact that said guiding grooves (21) are obtained on the outer surface of said first tubular body (11) and said guiding pin (22) is associated with the inner surface of said second tubular body (12).

10. Device (1) according to claim 8, **characterized by** the fact that said guiding grooves (21) are obtained on the inner surface of said second tubular body (12) and said guiding pin (22) is associated with the outer surface of said first tubular body (11).

11. Device (1) according to one or more of claims 8 to 10, **characterized by** the fact that said guiding grooves (21) are substantially arranged one alongside the other.

12. Device (1) according to one or more of claims 8 to 11, **characterized by** the fact that said tubular bodies (11, 12) have a round section and can rotate around their own axis to force the passage of said guiding pin (22) from one guiding groove (21) to the other.

13. Device (1) according to one or more of the preceding claims, **characterized by** the fact that said return flexible means (26) comprise at least a section of said first tubular body (11) which has a helical-spring shape and an extremity stopped up against a protrusion (27) of said second tubular body (12).

14. Device (1) according to one or more of the preceding claims, **characterized by** the fact that said return flexible means (26) comprise at least a section of said second tubular body (12) which has a helical-spring shape and an extremity stopped up against a protrusion (27) of said first tubular body (11).

15. Device (1) according to one or more of the preceding claims, **characterized by** the fact that said contact element (12) comprises at least an edge (19, 20) suitable for coming into contact with the skin (P) of the user which has at least a sharp-edge section (20).

## Patentansprüche

1. Vorrichtung (1) zur Steuerung der Einstichtiefe einer Nadel für Injektionen, umfassend:
- mindestens ein Trägerelement (11), das mit mindestens einem von einer Abgabevorrichtung (2) zur Abgabe einer Substanz (F) und einer Injektionsnadel (3) für das Injizieren der Substanz (F) in die Haut (P) eines Benutzers verbindbar ist;
- mindestens ein Kontaktelement (12), das mit dem Trägerelement (11) in einer translatierbaren Art und Weise entlang einer Gleitrichtung (D) im Wesentlichen parallel zu der Injektionsnadel (3) verbindbar ist und das geeignet ist, mit der Haut (P) des Benutzers in Berührung zu kommen; und
- Einstellmittel (21, 22) für das Einstellen des Betätigungswegs von dem Kontaktelement (12) an dem Trägerelement (11),
**dadurch gekennzeichnet, dass** das Trägerelement (11) Verriegelungsmittel (17) der Injektionsnadel (3) aufweist, die mindestens ein Zahnelement aufweisen, welches mit einem entsprechenden Positionierungselement, das an der Injektionsnadel (3) ausgebildet ist, verriegelt werden kann, wobei das Trägerelement (11) vorgesehen ist, um an der Injektionsnadel (3) angebracht zu werden, die wiederum über der Abgabevorrichtung (2) angebracht ist.

2. Vorrichtung (1) gemäß Anspruch 1, **dadurch gekennzeichnet, dass** der Betätigungsweg zwischen einer ersten Wegendposition, welche der maximalen Längenausdehnung des Kontaktelements (12) an dem Trägerelement (11) entspricht, und einer zweiten Wegendposition definiert ist, welche der minimalen Längenausdehnung des Kontaktelements (12) an dem Trägerelement (11) entspricht, wobei die Einstellmittel (21, 22) zum Modifizieren der zweiten Wegendposition geeignet sind.

3. Vorrichtung (1) gemäß Anspruch 2, **dadurch gekennzeichnet, dass** die Einstellmittel (21, 22) elastische Rückstellmittel (26) umfassen, die zum Drücken des Kontaktelements (12) geeignet sind, damit es in die erste Wegendposition zurückkehrt.

4. Vorrichtung (1) gemäß Anspruch 2 oder 3, **dadurch gekennzeichnet, dass** das Trägerelement (11) mindestens einen ersten Positionierungszahn (23) aufweist und das Kontaktelement (12) mindestens einen zweiten Positionierungszahn (24) aufweist, wobei die Positionierungszähne (23, 24) geeignet sind, in der ersten Wegendposition in Kontakt zu kommen und die vollständige Entfernung des Kontaktelements (12) von dem Trägerelement (11) zu verhindern.

5. Vorrichtung (1) gemäß einem oder mehreren der vorhergehenden Ansprüche, **dadurch gekennzeichnet, dass** das Trägerelement (11) einen ersten rohrförmigen Körper aufweist und das Kontaktelement (12) einen zweiten rohrförmigen Körper aufweist, wobei die rohrförmigen Körper (11, 12) teleskopartig ineinander eingepasst sind und mindestens teilweise die Injektionsnadel (3) umgeben.

6. Vorrichtung (1) gemäß Anspruch 5, **dadurch gekennzeichnet, dass** der erste rohrförmige Körper (11) einen ersten Abschnitt (13) aufweist, der in dem zweiten rohrförmigen Körper (12) eingepasst ist, und einen zweiten Abschnitt (14), der aus dem zweiten rohrförmigen Körper (12) austritt.

7. Vorrichtung (1) gemäß Anspruch 6, **dadurch gekennzeichnet, dass** der erste rohrförmige Körper (11) mindestens einen verbreiterten Manöverabschnitt (15) aufweist, der mit dem zweiten Abschnitt (14) verbunden ist.

8. Vorrichtung (1) gemäß einem oder mehreren der vorhergehenden Ansprüche, **dadurch gekennzeichnet, dass** die Einstellmittel (21, 22) umfassen:
- mindestens eine Gruppe von Führungsrillen (21), die auf einem von dem Trägerelement (11) und dem Kontaktelement (12) ausgebildet sind und sich im Wesentlichen parallel zu der Gleitrichtung (D) erstrecken; und
- mindestens einen Führungsstift (22), der mit dem anderen von dem Trägerelement (11) und dem Kontaktelement (12) verbunden ist und wahlweise in einer gleitenden Art und Weise in einer der Führungsrillen (21) angebracht werden kann;
wobei die Führungsrillen (21) unterschiedliche Längen aufweisen, um unterschiedliche Betätigungswege zu definieren.

9. Vorrichtung (1) gemäß Anspruch 8, **dadurch gekennzeichnet, dass** die Führungsrillen (21) auf der äußeren Oberfläche von dem ersten rohrförmigen Körper (11) erhalten werden und der Führungsstift (22) mit der inneren Oberfläche von dem zweiten rohrförmigen Körper (12) verbunden ist.

10. Vorrichtung (1) gemäß Anspruch 8, **dadurch gekennzeichnet, dass** die Führungsrillen (21) auf der inneren Oberfläche von dem zweiten rohrförmigen Körper (12) ausgebildet sind und der Führungsstift (22) mit der äußeren Oberfläche von dem ersten rohrförmigen Körper (12) verbunden ist.

11. Vorrichtung (1) gemäß einem oder mehreren der Ansprüche 8 bis 10, **dadurch gekennzeichnet, dass** die Führungsrillen (21) im Wesentlichen nebeneinander angeordnet sind.

12. Vorrichtung (1) gemäß einem oder mehreren der Ansprüche 8 bis 11, **dadurch gekennzeichnet, dass** die rohrförmigen Körper (11, 12) einen runden Bereich aufweisen und sich um ihre eigene Achse drehen können, um den Übergang von dem Führungsstift (22) von einer Führungsrille (21) zur anderen zu erzwingen.

13. Vorrichtung (1) gemäß einem oder mehreren der vorhergehenden Ansprüche, **dadurch gekennzeichnet, dass** die elastischen Rückstellmittel (26) mindestens einen Bereich des ersten rohrförmigen Körpers (11) umfassen, der eine Schraubenfederform und eine Extremität aufweist, die gegen einen Vorsprung (27) des zweiten rohrförmigen Körpers (12) anschlägt.

14. Vorrichtung (1) gemäß einem oder mehreren der vorhergehenden Ansprüche, **dadurch gekennzeichnet, dass** die elastischen Rückstellmittel (26) mindestens einen Bereich des zweiten rohrförmigen Körpers (11) umfassen, der eine Schraubenfederform und eine Extremität aufweist, die gegen einen Vorsprung (27) des ersten rohrförmigen Körpers (11) anschlägt.

15. Vorrichtung (1) gemäß einem oder mehreren der vorhergehenden Ansprüche, **dadurch gekennzeichnet, dass** das Kontaktelement (12) mindestens eine Kante (19, 20) aufweist, die geeignet ist, um mit der Haut (P) des Benutzers in Kontakt zu kommen und die die mindestens einen scharfkantigen Bereich (20) aufweist.

## Revendications

1. Dispositif (1) de contrôle de la profondeur de pénétration d'une aiguille d'injection, comprenant :
- au moins un élément de support (11), qui peut être associé à au moins un parmi un dispositif de distribution (2) pour l'administration d'une substance (F) et une aiguille d'injection (3) pour l'injection de la substance (F) dans la peau (P) d'un utilisateur ;
- au moins un élément de contact (12), qui peut être associé audit élément de support (11) de manière translationnelle selon une direction de coulissement (D) sensiblement parallèle à ladite aiguille d'injection (3) et apte à entrer en contact avec la peau (P) de l'utilisateur ; et
- des moyens d'ajustement (21, 22) pour ajuster la course de déplacement dudit élément de contact (12) sur ledit élément de support (11) ;
**caractérisé en ce que** ledit élément de support (11) comprend des moyens d'emboîtement (17) de ladite aiguille d'injection (3) qui comprennent au moins un élément de dent qui peut être emboîtée sur un positionneur correspondant obtenu sur ladite aiguille d'injection (3), ledit élément de support (11) est conçu pour être monté sur ladite aiguille d'injection (3), qui à son tour est montée pardessus ledit dispositif de distribution (2).

2. Dispositif (1) selon la revendication 1, **caractérisé en ce que** ladite course de déplacement est définie entre une première position de fin de course, correspondant à l'allongement maximal dudit élément de contact (12) sur ledit élément de support (11), et une seconde position de fin de course, correspondant à l'allongement minimal dudit élément de contact (12) sur ledit élément de support (11), lesdits moyens d'ajustement (21, 22) étant aptes à modifier ladite seconde position de fin de course.

3. Dispositif (1) selon la revendication 2, **caractérisé en ce que** lesdits moyens d'ajustement (21, 22) comprennent des moyens flexibles de rappel (26) aptes à pousser ledit élément de contact (12) pour revenir dans ladite première position de fin de course.

4. Dispositif (1) selon la revendication 2 ou 3, **caractérisé en ce que** ledit élément de support (11) comprend au moins une première dent de positionnement (23) et ledit élément de contact (12) comprend au moins une seconde dent de positionnement (24), lesdites dents de positionnement (23, 24) étant aptes à entrer en contact dans ladite première position de fin de course et empêcher le retrait complet dudit élément de contact (12) dudit élément de support (11).

5. Dispositif (1) selon l'une ou plusieurs des revendications précédentes, **caractérisé en ce que** ledit élément de support (11) comprend un premier corps tubulaire et ledit élément de contact (12) comprend un second corps tubulaire, dans lequel lesdits corps tubulaires (11, 12) sont montés téléscopiquement l'un à l'intérieur de l'autre et entourent au moins partiellement ladite aiguille d'injection (3).

6. Dispositif (1) selon la revendication 5, **caractérisé en ce que** ledit premier corps tubulaire (11) comprend une première portion (13) agencée dans ledit second corps tubulaire (12) et une seconde portion (14) sortant dudit second corps tubulaire (12).

7. Dispositif (1) selon la revendication 6, **caractérisé en ce que** ledit premier corps tubulaire (11) comprend au moins une portion de manoeuvre élargie (15) associée à ladite seconde portion (14).

8. Dispositif (1) selon l'une ou plusieurs des revendications précédentes, **caractérisé en ce que** lesdits moyens d'ajustement (21, 22) comprennent :
- au moins un groupe de rainures de guidage (21), qui sont obtenues sur un parmi ledit élément de support (11) et ledit élément de contact (12) et s'étendent sensiblement parallèles à ladite direction de coulissement (D) ; et
- au moins un pion de guidage (22), qui est associé à l'autre parmi ledit élément de support (11) et ledit élément de contact (12) et peut être agencé, de manière alternative, coulissant dans l'une desdites rainures de guidage (21) ;
lesdites rainures de guidage (21) ayant des longueurs différentes pour définir des courses de déplacement différentes.

9. Dispositif (1) selon la revendication 8, **caractérisé en ce que** lesdites rainures de guidage (21) sont obtenues sur la surface extérieure dudit premier corps tubulaire (11) et ledit pion de guidage (22) est associé à la surface intérieure dudit second corps tubulaire (12).

10. Dispositif (1) selon la revendication 8, **caractérisé en ce que** lesdites rainures de guidage (21) sont obtenues sur la surface intérieure dudit second corps tubulaire (12) et ledit pion de guidage (22) est associé à la surface extérieure dudit premier corps tubulaire (11).

11. Dispositif (1) selon l'une ou plusieurs des revendications 8 à 10, **caractérisé en ce que** lesdites rainures de guidage (21) sont sensiblement agencées l'une à côté de l'autre.

12. Dispositif (1) selon l'une ou plusieurs des revendications 8 à 11, **caractérisé en ce que** lesdits corps tubulaires (11, 12) présentent une section ronde et peuvent tourner en rotation autour de leur propre axe pour forcer le passage dudit pion de guidage (22) d'une rainure de guidage (21) à l'autre.

13. Dispositif (1) selon l'une ou plusieurs des revendications précédentes, **caractérisé en ce que** lesdits moyens flexibles de rappel (26) comprennent au moins une section dudit premier corps tubulaire (11) qui présente une forme de ressort hélicoïdal et une extrémité agencée en butée contre une protubérance (27) dudit second corps tubulaire (12).

14. Dispositif (1) selon l'une ou plusieurs des revendications précédentes, **caractérisé en ce que** lesdits moyens flexibles de rappel (26) comprennent au moins une section dudit second corps tubulaire (12) qui présente une forme de ressort hélicoïdal et une extrémité agencée en butée contre une protubérance (27) dudit premier corps tubulaire (11).

15. Dispositif (1) selon l'une ou plusieurs des revendications précédentes, **caractérisé en ce que** ledit élément de contact (12) comprend au moins un bord (19, 20) apte à entrer en contact avec la peau (P) de l'utilisateur qui possède au moins une section à bord vif (20).
